# EUROPEAN PATENT SPECIFICATION

(11) **EP 3 348 234 B1**
(45) Date of publication and mention of the grant of the patent: **22.01.2025**
(21) Application number: 18151017.3
(22) Date of filing: 10.01.2018
(51) Int. Cl.: A61F 2/12, A61L 27/56, C08J 9/36

(54) **INTERNAL MAMMARY PROSTHESIS MADE OF POLYMER FOAM MATERIAL FOR RECONSTRUCTIVE SURGERY**
AUS POLYMERSCHAUMSTOFFMATERIAL HERGESTELLTE, INTERNE BRUSTPROTHESE ZUR REKONSTRUKTIVEN CHIRURGIE
PROTHÈSE MAMMAIRE INTERNE CONSTITUÉE D'UN MATÉRIAU DE MOUSSE POLYMÈRE POUR CHIRURGIE RÉPARATRICE

(30) Priority: 11.01.2017 IT 201700002146
(43) Date of publication of application: 18.07.2018
(73) Proprietor: Leo Holding S.A., 6948 Porza (CH)
(72) Inventor: FENAROLI, Privato, 3005 BERN (CH)
(74) Representative: Gualeni, Nadia

(56) References cited:
- WO-A1-2014/118773
- WO-A2-03/017868
- GB-A- 2 040 688
- US-A- 3 366 975
- US-A- 4 767 794
- US-A1- 2003 093 151
- US-A1- 2006 036 333
- US-A1- 2016 082 235
- US-B1- 6 916 339

## Description

The object of the present invention is an internal mammary prosthesis for reconstructive surgery.

Furthermore, the object of the present invention is also a method of production of an internal mammary prosthesis.

As is known, reconstructive surgery is used to reconstruct parts of the body, for example after surgery for removal following pathologies or when such parts have been damaged by traumatic events. With particular reference to reconstructive surgery of the breast, the need to protect the physical integrity of women, considered important enough to drive plastic surgery to develop increasingly effective reconstruction techniques capable of repairing the extensive damages resulting from, for example, radical mastectomy, has now become well established.

With reference to breast reconstructive surgery, various types of internal mammary prostheses are known, for example anatomical prostheses (or teardrop prostheses) that allow a harmonious and natural breast to be created based on the shape of the rib cage, or round prostheses, which are very useful when a homogeneous increase in the volume of the breast without changing the shape thereof is desired.

Most of the known breast implants consist of an outer silicone casing inside of which is contained, for example, a cohesive silicone gel or a saline solution.

However, such known prostheses have some drawbacks, for example, those related to the risk of breakage of the outer casing and the resulting dispersion of filling liquid (in most cases, silicone).

Further drawbacks are related to the weight of the prosthesis, which contributes to aggravating the phenomenon of capsular contracture (or hardening). In fact, the organism reacts to the prosthesis like any other foreign body, giving rise to the formation of a fibrous "capsule". Normally, such "capsule" presents itself only as a thin containment film consisting of collagen. In some cases, because of the weight of the prosthesis, this capsule thickens and contracts making the breast hard with an unnatural appearance and consistency.

Because of all these drawbacks related to known mammary prostheses, new reconstructive surgeries are often required.

The need for a particularly lightweight internal mammary prosthesis is therefore felt in the field of reconstructive surgery.

Examples of known prostheses are described in documents WO03/017868, WO2014/118773, US4767794 and US3366975, that refer to prostheses having a silicone skin and a silicone foam body core, and in documents GB2040688, US2016/082235, US2003/093151 and US2006/036333, that refer to prostheses having an outer silicone casing inside of which is contained, for example, a cohesive silicone gel or a saline solution. Document US6916339 describes a prosthesis wherein the posterior surface has at least one concave curvature, to improve the fit of the prosthesis on the thorax.

The object of the present invention is to solve the problems of the prior art while taking into account the needs of the industry.

This object is achieved by an internal mammary prosthesis made of polymer foam material (or poly-foam material).

Advantageously, a prosthesis made in this way is particularly suitable for attachment in a supramuscular position.

Such object is achieved by an internal mammary prosthesis in accordance with claim 1 and in accordance with the related method of construction thereof according to claims 13 and 14. The dependent claims describe preferred or advantageous embodiments of the prosthesis.

The features and advantages of the mammary prosthesis and the method of construction thereof according to the present invention will be apparent from the description given hereinafter, provided by way of non-limiting example, in accordance with the accompanying figures, wherein:
- figure 1 shows a side sectional view of a mammary prosthesis implanted within the patient's body according to the present invention;
- figure 2 shows a top sectional view of a mammary prosthesis implanted within the patient's body according to the present invention;
- figure 3 shows a top sectional view of a mammary prosthesis according to the present invention;
- figure 4 shows a side sectional view of a mammary prosthesis according to the present invention;
- figure 5 shows a front view of a self-supporting prosthesis provided with an attachment portion in a variant embodiment;
- figure 6 shows a front view of a self-supporting prosthesis provided with an attachment portion, in a further variant embodiment.

With reference to the attached figures, an internal mammary prosthesis 100 is shown.

The mammary prosthesis 100 comprises a front portion 102, which constitutes the prosthesis portion suitable to fill the area wherein the prosthesis will be implanted, and a back 103, opposite the front portion 102.

The mammary prosthesis 100 comprises a solid body 10 made of a spongy material.

The body 10 is made of polymer foam material (or poly-foam) with a density ranging from 0.10 g/cm³ to 0.50 g/cm³. A mammary prosthesis made in this way is lighter than the known internal mammary prostheses made of a cohesive silicone gel.

Preferably, the poly-foam material is expanded silicone or silicone foam, or polyurethane foam.

Preferably, the body 10 is made of silicone foam with a density of between 0.15 g/cm³ and 0.35 g/cm³, preferably between 0.20 g/cm³ and 0.30 g/cm³.

In an alternative example, the body 10 is made of polyurethane foam with a density of between 0.10 g/cm³ and 0.30 g/cm³, preferably between 0.15 g/cm³ and 0.25 g/cm³.

Regardless of the poly-foam material used, the mammary prosthesis 100 is provided with a film (or coating), preferably of silicone. It should be noted that film refers to a thin plastic film. This film has a barrier function, i.e., it is suitable to make the spongy surface of the body 100 impermeable, so that such surface is suitable for implanting the prosthesis 100 inside the patient's body.

The silicone film is obtained by dipping, that is by immersing (preferably several times) the body 10 made of spongy material in suitable silicone solutions.

Preferably, the prosthesis 100 is provided with a certain surface roughness or undulation, that is to say that the surface finish of the self-supporting prosthesis is of the type known as textured. Such constructive choice allows the superficial adhesion of the tissues on the self-supporting prosthesis to be improved and facilitated once the latter has been implanted inside the patient's body.

Since the mammary prosthesis 100 has a solid body 10 made of spongy material, therefore less adaptable than traditional silicone gel prostheses, the shape of the prosthesis is particularly important to ensure the most natural effect possible and a positioning more adaptive to the body.

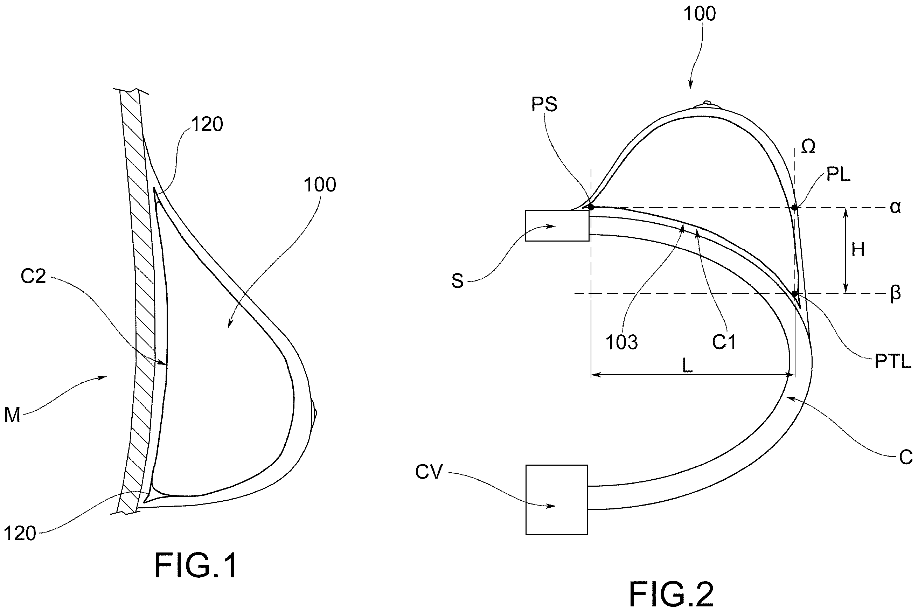

For example, the front portion 102 has a teardrop shape (as in figure 1).

Preferably, the mammary prosthesis 100 has an edge that tapers outwards, to better follow the natural shape of the mammary gland and to facilitate the positioning of the prosthesis under the muscle.

Preferably, moreover, the mammary prosthesis 100 has a geometry such as to take into account the irregularity of shape, volume and surface typical of the mammary gland of the patient to whom it will be applied. Such geometry is obtainable by using a three-dimensional (3D) mold that traces exactly the shape of the mammary gland portion that must be removed from the patient, to minimize or even avoid changes in the natural shape of the patient's breast. Preferably, therefore, such three-dimensional mold is obtained by means of a particular method described hereinafter.

With reference to figure 2, the prosthesis 100 implanted in the patient's body is shown. In particular, figure 2 shows a sectional view wherein is visible the sternum S, the right portion of the rib cage C, and the spinal column CV. Figure 2 also shows the sternal point PS (to be understood as the most medial point of the prosthesis) and the lateral thoracic point PTL (to be understood as the most lateral point of the prosthesis).

The points PS and PTL delimit the base of the prosthesis.

α is defined as the plane passing through the point PS and parallel to the sternum S, and β is defined as the plane passing through the point PTL and parallel to the sternum S, the height H is defined as the distance between the planes α and β.

Moreover, Ω is defined as the plane passing through the point PTL and orthogonal to the sternum S; the intersection between the planes α and Ω defines the point PL.

The length L is defined as the distance between the points PS and PL.

Along the plane α are defined the points L_{1/3}, at a distance equal to one third of the distance L calculated from point PS, and L_{2/3}, at a distance equal to two thirds of the distance L calculated from the point PS.

Along the plane Ω are defined the points H_{1/3} at a height equal to one third of the height H calculated starting from the point PL, and H_{2/3} at a height equal to two thirds of the height H calculated starting from the point PL.

Furthermore, Δ defines the plane passing through point L_{2/3} and orthogonal to the plane α, and µ the plane passing through point H_{1/3} and orthogonal to the plane Ω.

The reference points P_{1/3} and P_{2/3} are thus defined.

Point P_{1/3} corresponds to point L_{1/3}.

Point P_{2/3} is given by the intersection of the planes Δ and µ.

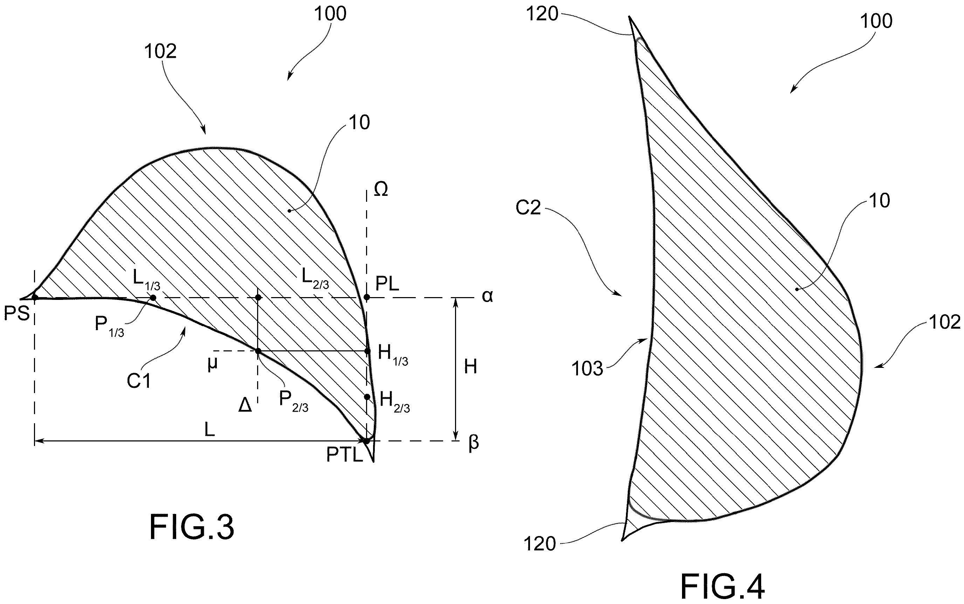

The prosthesis 100 is therefore provided, at the back 103 and in cross section (figure 3), with a particular curvature C1.

Such curvature C1 is obtained by interpolating at least the points PS, P_{2/3} and PTL.

Preferably the curvature C1 is obtained by interpolating all the points PS, P_{1/3}, P_{2/3} and PTL.

Such curvature C1 allows the back 103 of the prosthesis 100 to follow as much as possible the natural curvature of the patient's chest.

The prosthesis 100 is also provided, at the back 103 and in longitudinal section (figure 4), with a slight curvature C2, or a slight recess, to follow as much as possible the natural curvature of the patient's chest. That is, the back 103 of the prosthesis 100 is slightly concave.

It is therefore important to provide the prosthesis 100 with a back 103 with a recess adapted to follow as much as possible the natural curvature of the patient's chest.

Advantageously, the prosthesis 100 is insertable interlocking under the skin of the patient and kept in position by the skin itself only by virtue of the lightness of the prosthesis (the body 10 is made of polymer foam material with a density ranging from 0.10 g/cm³ to 0.50 g/cm³), of its shape (curvature C1 that allows the back 103 of the prosthesis 100 to follow as much as possible the natural curvature of the patient's chest) and its volume (reproducing the volume of the removed mammary gland), and by the fact that being solid (solid body 10 made of spongy material) there are no internal movements of liquid/gel to be controlled. Advantageously, the presence of edges which taper outwards facilitates the interlocking attachment, allowing the prosthesis to remain in position without the need for sewing.

In a variant embodiment, the body 10 is also provided with an attachment portion 120 suitable to allow the prosthesis to be fixed directly on the muscular tissue of the area wherein the prosthesis will be implanted. That is to say, the attachment portion 120 constitutes a support for sewing, and in particular for sutures, of the prosthesis 100 directly onto the muscular tissue.

In particular, the attachment portion 120 is an integral part of the body 10, i.e. it is made in one piece therewith and is made of the same material.

The attachment portion 120 protrudes externally with respect to the body 10.

The attachment portion 120 is positioned between the back 103 and the front portion 102.

The attachment portion 120 lies on the same plane as the back 103, i.e. it is coplanar with the back 103. That is, the attachment portion 120 is an extension of the back 103, which projects at least partially outwardly with respect to the front portion 102.

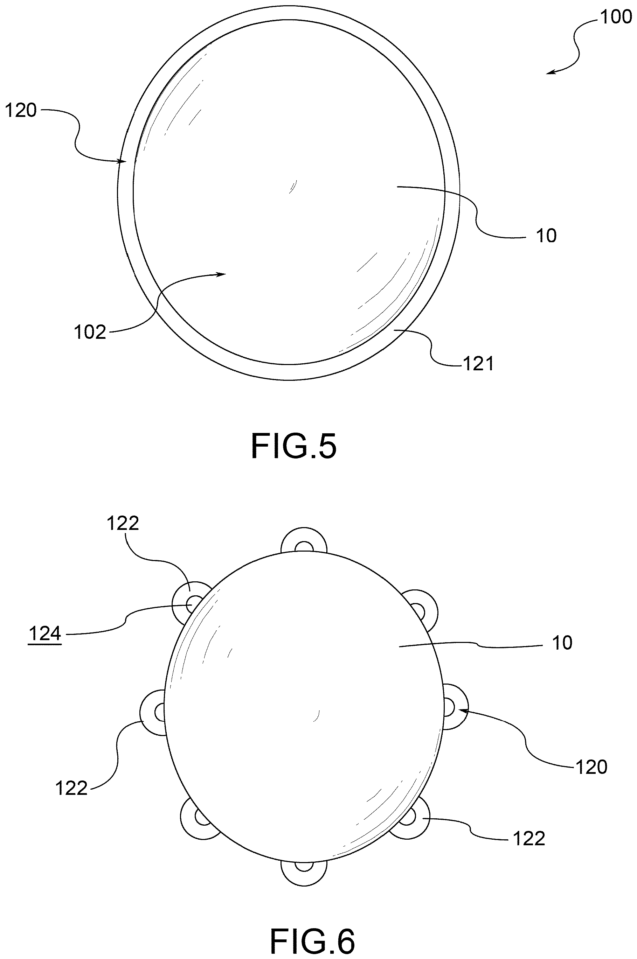

In the embodiment of figure 5, the attachment portion 120 is a continuous element arranged around the body 10. Preferably, the attachment portion 120 is a continuous annular edge 121. Such constructive choice allows greater freedom when fixing the self-supporting prosthesis, as it is possible to make a suture at any point of the profile of the body 10.

The attachment portion 120 is therefore suitable to be perforated and penetrated by a needle or in general by a pointed element to allow the prosthesis, and therefore the body 10, to be stitched directly onto the muscular tissue of the area wherein the prosthesis will be implanted.

Preferably, the attachment portion 120 is continuous with respect to the body 10 to ensure continuity in the shape and geometry of the prosthesis itself (i.e. between the body 10 and the attachment portion 120 there are no steps or discontinuities).

Preferably, moreover, the attachment portion 120 is an edge that tapers outwards to better follow the natural shape of the mammary gland and to facilitate the positioning under the muscle of the prosthesis.

In the variant of figure 6, the attachment portion 120 comprises a plurality of slots 122, uniformly distributed, each defining a hole 124 (or an opening or a passage) for the passage of the suture thread.

In such variant embodiment, therefore, being that the mammary prosthesis 100 is composed of a solid body 10 made of lightweight, spongy material and provided with an attachment portion 120, such prosthesis may be sewn directly onto the muscular tissue of the area wherein the prosthesis will be implanted.

### EXAMPLE WITH EXPANDED SILICONE (OR SILICONE FOAM).

In such example embodiment, the prosthesis 100, and in particular the body 101, is made of a two-component silicone foam. The catalysis process by addition takes place by means of a specific platinum or palladium catalyst which gives the compound biocompatibility characteristics.

The expansion is activated at room temperature.

The calculation of the dosage of components A and B, expressed in volume (V_{A} and V_{B}), is carried out as a ratio of the final volume V (V_{F}) of the prosthesis 100 to be obtained. Preferably, the dosage is calculated as: V_{A} + V_{B} = 20% V_{F}

Considering that the two components (A and B) have a mixing ratio of 1:1, the dosage is calculated as:
VA = 10% VF and VB = 10% VF

For example, to make a prosthesis (and therefore to recreate a breast) with a 400 ml volume, 40 ml of component A and 40 ml of component B must be combined, for a total of 80 ml (sum of components A + B), which represents precisely 20% of the final volume of the prosthesis.

In this example, the density of the silicone foam is between 0.20 g/cm³ and 0.35 g/cm³.

The method of making the prosthesis 100 provides for the use of a three-dimensional (3D) mold, preferably a mold that exactly traces the shape of the mammary gland portion that must be removed from the patient.

The mold may be, for example, of the open type, i.e., comprising a female portion closed by a cover, said female portion defining therein the cavity of the mold.

Alternatively, the mold may be, for example, of the closed type, i.e., comprising a female portion provided with a hole for feeding the material, said female portion defining therein the cavity of the mold.

The method of construction of the prosthesis 100 provides for the step of combining in the mold cavity at room temperature the components A and B, and mixing, preferably quickly, to avoid the formation of bubbles.

If an open mold is used, the method then provides, when the expansion volume of the silicone foam reaches about 2/3 of the total volume of the mold cavity, for closing the mold with the cover.

The method then provides for keeping the mold closed until the complete formation of the spongy body 101, for example, after 25 minutes.

The method also provides for waiting for the silicone foam to stabilize before removing the spongy body 101 from the mold, for example, after another 60 minutes.

At this point, a silicone coating for closing the pores is required for the spongy body 101. The method of making the prosthesis 100 therefore provides for a step of covering the body 101 with at least one layer, preferably several layers, of silicone to form a thin film on the spongy body 101 of the prosthesis. Such operation provides for dipping the spongy body 101 in the liquid silicone and drying at room temperature; this operation may be repeated several times, until the desired thickness of the silicone cover is obtained.

Finally, the method provides for the sterilization step of the prosthesis 100, for example in ethylene oxide.

### EXAMPLE WITH POLYURETHANE FOAM.

Polyurethane is obtained by reaction between a diisocyanate (aromatic or aliphatic) and a polyol (e.g. a poly-propylene glycol or a polyester-diol).

The reaction is carried out in the presence of catalysts to increase the speed of the reaction, and other additives, to impart certain characteristics to the material to be obtained. In the example embodiment, a surfactant is used to lower the surface tension and thus promote the formation of the foam.

In particular, for the device in question, crosslinked polyurethane foams, and therefore biostable, with a high percentage of open porosity, may be used, using a suitable mixture of polyether polyols and catalysts able to obtain high open porosity and adequate values of rigidity and mechanical strength.

The isocyanate, which makes it possible to obtain the crosslinking bridges with the side groups of the polyols, is, for example, diphenylmethane diisocyanate (polymeric MDI) .

The polyurethane foam is obtained by gas foaming using water as an expanding agent in an amount varying from 0.5 to 10% as a percentage by weight W/W of the polyol.

In such example, the density of the expanded polyurethane is between 0.15 g/cm³ and 0.25 g/cm³.

Also in this case, the method of making the prosthesis 100 therefore provides a step of covering the body 101 with at least one layer, preferably several layers, of silicone to form a thin film on the spongy body 101 of the prosthesis. Such operation provides for dipping the spongy body 101 in the liquid silicone and drying at room temperature; this operation may be repeated several times, until the desired thickness of silicone cover is obtained.

Finally, the method provides for the sterilization step of the prosthesis 100, for example in ethylene oxide.

It is also described a method of construction of a custom-made prosthesis 100, i.e. a prosthesis provided with a shape and a volume corresponding to the portion of mammary gland that must be removed from the patient. Advantageously, therefore, the internal mammary prosthesis 100 traces the uniqueness of the mammary gland to be replaced.

In order to obtain the unique shape of the mammary gland to be replaced, it is necessary to define the patient's mammary gland in three dimensions (i.e. in 3D). The method therefore provides for:
a. Collecting digital data on the shape and external dimensions of the original mammary gland by means of scanning;
b. Collecting digital data by CT (Computerized Axial Tomography) or Micro-CT, on the shape and dimensions of the support base, of the rib cage, of the original mammary gland;
c. processing (via software) the digital data collected in steps a and b to define three-dimensionally the shape, size and volume of the body 10 of the internal mammary prosthesis 100 which will replace (by interlocking or by sewing) the original mammary gland removed surgically.

For example, step a) provides for calculating the volume of the patient's mammary gland, for example by breast scanner, ultrasound to measure the thickness of the skin and the depth of the mammary gland.

The method of making the custom prosthesis therefore provides for creating a three-dimensional mold based on the specific measurements of the patient's breast, and in particular the portion of the mammary gland that must be removed from the patient, in order to minimize or even avoid changes in the natural shape of the patient's breast.

The mold may be, for example, of the open type or of the closed type.

The mold may be made, for example, by three-dimensional printers or by methods of material removal (for example, by milling).

It is also described an implantation method of the mammary prosthesis 100.

The implantation method of the mammary prosthesis 100 provides for the steps of:
- positioning the prosthesis against the pectoralis major muscle M;
- attaching the prosthesis to the pectoralis major muscle M.

In an example embodiment, the prosthesis 100 is provided with an attachment portion 120, for example in the form of an externally protruding edge (between the front portion 102 and the back 103), and the attachment step provides for sewing the attachment portion 120 directly to the muscular tissue via a suture stitch.

Innovatively, a mammary prosthesis according to the present invention is particularly lightweight compared to traditional silicone implants.

Advantageously, such a lightweight mammary prosthesis may be implanted directly on the muscle tissue, reducing post-operative recovery times and the risks of relocation.

Advantageously, a mammary prosthesis according to the present invention avoids the typical drawbacks of known prostheses, such as the risk of dispersion of filling liquid.

Advantageously, a mammary prosthesis is provided with a shape that follows the portion of the mammary gland that must be removed from the patient, in order to avoid any change in the natural shape of the patient's breast.

It is clear that one skilled in the art may make modifications to the above-described product, all contained within the scope of protection as defined by the following claims.

## Claims

1. An internal mammary prosthesis (100), implantable inside the body of the patient in reconstructive surgery, has a solid body (10) of polymer foam material having a density between 0.10 g/cm³ and 0.50 g/cm³ so as to be particularly lightweight, the body (10) being provided with a coating and comprising a front portion (102) and a back (103), and having defined, on a cross-section of the prosthesis in a transverse plane virtually sectioning the sternum of a patient when the prosthesis is in use:
- the sternal point (SP), at the most medial point of the prosthesis, and the lateral thoracic point (PTL), at the most lateral point of the prosthesis, the sternal point (SP) and the lateral thoracic point (PTL) delimiting the base of the prosthesis;
- a plane α passing through the sternal point (SP) and parallel to the sternum (S);
- a plane β passing through the lateral thoracic point (PTL) and parallel to the sternum (S);
- the height (H) as the distance between the planes α and β;
- a plane Ω passing through the lateral thoracic point (PTL) and orthogonal to the sternum (S);
- the point of intersection (PL) between the planes α and Ω;
- the length (L) as the distance between the sternal point (SP) and the point of intersection (PL);
- the points L_{1/3}, at a distance equal to one third of the length (L) calculated starting from the sternal point (SP), and L_{2/3}, at a distance equal to two thirds of the length (L) calculated starting from the sternal point (SP);
- along the plane Ω, the points H_{1/3} at a height equal to one third of the height (H) calculated starting from the point of intersection (PL), and H_{2/3}, at a height equal to two thirds of the height (H) calculated starting from the point of intersection (PL);
- the plane Δ passing through the point L_{2/3} and orthogonal to the plane α;
- the plane µ passing through the point H_{1/3} and orthogonal to the plane Ω;
- the reference points P_{1/3} as corresponding to L_{1/3}, and P_{2/3} as given by the intersection between the planes Δ and µ;
the internal mammary prosthesis being **characterized in that** the the back (103) of the body (10) has, in cross-section, a curvature (C1) obtained by interpolating at least the sternal point (SP), the reference point P_{2/3} and the lateral thoracic point (PTL).

2. Internal mammary prosthesis (100) according to claim 1, wherein the polymer foam material is silicone foam with a density between 0.15 g/cm³ and 0.35 g/cm³.

3. Internal mammary prosthesis (100) according to claim 2, wherein the silicone foam is a two-component silicone foam with a platinum catalyst, wherein the dosage of components (A, B) expressed in volume (V_{A}, V_{B}) depends on the final volume (V_{F}) of the prosthesis (100).

4. Internal mammary prosthesis (100) according to claim 1, wherein the polymer foam material is polyurethane foam with density between 0.10 g/cm³ and 0.30 g/cm³.

5. Internal mammary prosthesis (100) according to any of the preceding claims, provided with an edge between the back (103) and the front portion (102) that tapers outwards.

6. Internal mammary prosthesis (100) according to any of the claims from 1 to 4, wherein the body (10) is provided with an attachment portion (120) which constitutes a support for sewing the prosthesis (100) directly onto the muscle tissue.

7. Internal mammary prosthesis (100) according to claim 6, in which the attachment portion (120) is an integral part of the body (10), made in one piece therewith and made of the same material.

8. Internal mammary prosthesis (100) according to claim 6 or 7, wherein the attachment portion (120) is a continuous annular edge (121) arranged around the body (10).

9. Internal mammary prosthesis (100) according to any of the preceding claims, wherein the attachment portion (120) is an edge that tapers outwards, to better follow the natural shape of the mammary gland and to facilitate the positioning of the prosthesis under the muscle.

10. Internal mammary prosthesis (100) according to claim 6 or 7 or 9, wherein the attachment portion (120) comprises a plurality of slots (122), uniformly distributed, each defining a hole (124) for the passage of a suture thread.

11. Internal mammary prosthesis (100) according to any of the preceding claims, wherein the back (103) has, in cross-section, a curvature (C1) obtained by interpolating the sternal point (SP), the reference point P1/3, the reference point P_{2/3} and the lateral thoracic point (PTL).

12. Internal mammary prosthesis (100) according to any of the preceding claims, wherein the coating is in silicone.

13. Method of making an internal mammary prosthesis (100) according to claim 3, comprising the steps of:
- preparing a mold comprising a female portion and a lid, said female portion defining within it the cavity of the mold;
- combining in the cavity of the mold, at room temperature, the components (A, B) and mixing;
- allowing the silicon foam to expand until the volume reaches 2/3 of the total volume of the mold cavity, and then closing the cavity with the lid.

14. Method of making an internal mammary prosthesis (100) according to claim 13, wherein the step of providing a mold comprises the steps of:
a. collecting digital data on the shape and external dimensions of the mammary gland of the patient using mammary gland scanning;
b. collecting digital data on the size and shape of the ribcage and the mammary gland of the patient by CT scan (computerized axial tomography) or Micro-CT;
c. processing using software the digital data collected in steps a) and b) to define three-dimensionally the shape, size and volume of the mold cavity.

## Patentansprüche

1. Innenbrustprothese (100), die in der rekonstruktiven Chirurgie in den Körper der Patientin implantierbar ist, aufweisend einen festen Körper (10) aus Polymerschaummaterial mit einer Dichte zwischen 0,10 g/cm³ und 0,50 g/cm³, um besonders leicht zu sein, wobei der Körper (10) mit einer Beschichtung versehen ist und einen vorderen Abschnitt (102) und eine Rückseite (103) umfasst und auf bzw. an einem Querschnitt der Prothese in einer Querebene, die das Sternum einer Patientin virtuell schneidet, wenn die Prothese verwendet wird, definiert aufweist:
- den Sternalpunkt (SP) an dem medialsten Punkt der Prothese und den lateralen Thoraxpunkt (PTL) an dem lateralsten Punkt der Prothese, wobei der Sternalpunkt (SP) und der laterale Thoraxpunkt (PTL) die Basis der Prothese begrenzen;
- eine Ebene α, die durch den Sternalpunkt (SP) und parallel zu dem Sternum (S) verläuft;
- eine Ebene β, die durch den lateralen Thoraxpunkt (PTL) und parallel zu dem Sternum (S) verläuft;
- die Höhe (H) als der Abstand zwischen den Ebenen α und β;
- eine Ebene Ω, die durch den lateralen Thoraxpunkt (PTL) und orthogonal zu dem Sternum (S) verläuft;
- den Schnittpunkt (PL) zwischen den Ebenen α und Ω;
- die Länge (L) als der Abstand zwischen dem Sternalpunkt (SP) und dem Schnittpunkt (PL);
- die Punkte L_{1/3} in einem Abstand, der gleich einem Drittel der Länge (L) berechnet beginnend ab dem Sternalpunkt (SP) ist, und L_{2/3} in einem Abstand, der gleich zwei Dritteln der Länge (L) berechnet beginnend ab dem Sternalpunkt (SP) ist;
- entlang der Ebene Ω die Punkte H_{1/3} in einer Höhe, die gleich einem Drittel der Höhe (H) berechnet beginnend ab dem Schnittpunkt (PL) ist, und H_{2/3} in einer Höhe, die gleich zwei Dritteln der Höhe (H) berechnet beginnend ab dem Schnittpunkt (PL) ist;
- die Ebene Δ, die durch den Punkt L_{2/3} und orthogonal zu der Ebene α verläuft;
- die Ebene µ, die durch den Punkt H_{1/3} und orthogonal zu der Ebene Ω verläuft;
- die Referenzpunkte P_{1/3}, die L_{1/3} entsprechen, und P_{2/3}, die durch den Schnittpunkt zwischen den Ebenen Δ und µ gegeben sind;
wobei die Innenbrustprothese **dadurch gekennzeichnet ist, dass** die Rückseite (103) des Körpers (10) im Querschnitt eine Krümmung (C1) aufweist, die durch Interpolieren von zumindest dem Sternalpunkt (SP), dem Referenzpunkt P_{2/3} und dem lateralen Thoraxpunkt (PTL) erhalten wird.

2. Innenbrustprothese (100) nach Anspruch 1, wobei das Polymerschaummaterial Silikonschaum mit einer Dichte zwischen 0,15 g/cm³ und 0,35 g/cm³ ist.

3. Innenbrustprothese (100) nach Anspruch 2, wobei der Silikonschaum ein Zweikomponenten-Silikonschaum mit einem Platinkatalysator ist, wobei die Dosierung von Komponenten (A, B), ausgedrückt in Volumen (V_{A}, V_{B}), von dem endgültigen Volumen (V_{F}) der Prothese (100) abhängt.

4. Innenbrustprothese (100) nach Anspruch 1, wobei das Polymerschaummaterial Polyurethanschaum mit einer Dichte zwischen 0,10 g/cm³ und 0,30 g/cm³ ist.

5. Innenbrustprothese (100) nach einem der vorhergehenden Ansprüche, bereitgestellt bzw. versehen mit einem Rand zwischen der Rückseite (103) und dem vorderen Abschnitt (102), der sich nach außen verjüngt.

6. Innenbrustprothese (100) nach einem der Ansprüche 1 bis 4, wobei der Körper (10) mit einem Anbringungsabschnitt (120) versehen ist, der eine Stütze bzw. einen Träger zum Annähen der Prothese (100) direkt auf das Muskelgewebe bildet bzw. darstellt.

7. Innenbrustprothese (100) nach Anspruch 6, wobei der Anbringungsabschnitt (120) ein integraler Teil des Körpers (10) ist, einstückig mit diesem hergestellt ist und aus demselben Material besteht.

8. Innenbrustprothese (100) nach Anspruch 6 oder 7, wobei der Anbringungsabschnitt (120) ein durchgehender ringförmiger Rand (121) ist, der um den Körper (10) herum angeordnet ist.

9. Innenbrustprothese (100) nach einem der vorhergehenden Ansprüche, wobei der Anbringungsabschnitt (120) ein Rand ist, der sich nach außen verjüngt, um der natürlichen Form der Brustdrüse besser zu folgen und die Positionierung der Prothese unter dem Muskel zu erleichtern.

10. Innenbrustprothese (100) nach Anspruch 6 oder 7 oder 9, wobei der Anbringungsabschnitt (120) eine Mehrzahl von gleichmäßig verteilten Schlitzen (122) umfasst, die jeweils ein Loch (124) für den Durchgang eines Nahtfadens definieren.

11. Innenbrustprothese (100) nach einem der vorhergehenden Ansprüche, wobei die Rückseite (103) im Querschnitt eine Krümmung (C1) aufweist, die durch Interpolieren des Sternalpunkts (SP), des Referenzpunkts P_{1/3}, des Referenzpunkts P_{2/3} und des lateralen Thoraxpunkts (PTL) erhalten wird.

12. Innenbrustprothese (100) nach einem der vorhergehenden Ansprüche, wobei die Beschichtung Silikon ist.

13. Verfahren zum Herstellen einer Innenbrustprothese (100) nach Anspruch 3, umfassend die Schritte:
- Präparieren einer Form, die einen weiblichen bzw. aufnehmenden Abschnitt und einen Deckel umfasst, wobei der aufnehmende Abschnitt in seinem Inneren den Hohlraum der Form definiert;
- Kombinieren in dem Hohlraum der Form bei Raumtemperatur der Komponenten (A, B) und Mischen;
- Ausdehnenlassen des Silikonschaums, bis das Volumen 2/3 des Gesamtvolumens des Formhohlraums erreicht, und anschließendes Verschließen des Hohlraums mit dem Deckel.

14. Verfahren zum Herstellen einer Innenbrustprothese (100) nach Anspruch 13, wobei der Schritt des Bereitstellens einer Form die Schritte umfasst:
a. Erfassen von digitalen Daten über die Form und externe Abmessungen der Brustdrüse der Patientin unter Verwendung von Brustdrüsenscannen;
b. Erfassen von digitalen Daten über die Größe und Form des Brustkorbs und der Brustdrüse der Patientin durch CT-Scan (computergestützte Axialtomographie) oder Mikro-CT;
c. Verarbeiten, unter Verwendung von Software, der in den Schritten a) und b) erfassten digitalen Daten, um Form, Größe und Volumen des Formhohlraums dreidimensional zu definieren.

## Revendications

1. Prothèse mammaire interne (100), implantable à l'intérieur du corps de la patiente en chirurgie reconstructive, comportant un corps solide (10) en matériau de mousse polymère présentant une densité comprise entre 0,10 g/cm³ et 0,50 g/cm³ de manière à être particulièrement légère, le corps (10) étant pourvu d'un revêtement et comprenant une partie avant (102) et un arrière (103), et ayant défini, sur une section transversale de la prothèse dans un plan transversal coupant virtuellement le sternum d'une patiente lorsque la prothèse est en cours d'utilisation :
- le point sternal (SP), au niveau du point le plus interne de la prothèse, et le point thoracique latéral (PTL), au niveau du point le plus latéral de la prothèse, le point sternal (SP) et le point thoracique latéral (PTL) délimitant la base de la prothèse ;
- un plan α passant par le point sternal (SP) et parallèle au sternum (S) ;
- un plan β passant par le point thoracique latéral (PTL) et parallèle au sternum (S) ;
- la hauteur (H) en tant que distance entre les plans α et β ;
- un plan Ω passant par le point thoracique latéral (PTL) et perpendiculaire au sternum (S) ;
- le point d'intersection (PL) entre les plans α et Ω ;
- la longueur (L) en tant que distance entre le point sternal (SP) et le point d'intersection (PL) ;
- les points L_{1/3}, à une distance égale à un tiers de la longueur (L) calculée à partir du point sternal (SP), et L_{2/3}, à une distance égale à deux tiers de la longueur (L) calculée à partir du point sternal (SP) ;
- le long du plan Ω, les points H_{1/3} à une hauteur égale à un tiers de la hauteur (H) calculée à partir du point d'intersection (PL), et H_{2/3}, à une hauteur égale à deux tiers de la hauteur (H) calculée à partir du point d'intersection (PL) ;
- le plan Δ passant par le point L_{2/3} et perpendiculaire au plan α ;
- le plan µ passant par le point H_{1/3} et perpendiculaire au plan Ω ;
- les points de référence P_{1/3}, tel que correspondant à L_{1/3}, et P_{2/3},tel que donné par l'intersection entre les plans Δ et µ ;
la prothèse mammaire interne étant **caractérisée en ce que** l'arrière (103) du corps (10) présente, en section transversale, une courbure (C1) obtenue par interpolation au moins du point sternal (SP), du point de référence P_{2/3} et du point thoracique latéral (PTL).

2. Prothèse mammaire interne (100) selon la revendication 1, dans laquelle le matériau de mousse polymère est une mousse de silicone présentant une densité comprise entre 0,15 g/cm³ et 0,35 g/cm³.

3. Prothèse mammaire interne (100) selon la revendication 2, dans laquelle la mousse de silicone est une mousse de silicone à deux composants avec un catalyseur au platine, le dosage des composants (A, B), exprimé en volume (V_{A}, V_{B}), dépendant du volume final (V_{F}) de la prothèse (100).

4. Prothèse mammaire interne (100) selon la revendication 1, dans laquelle le matériau de mousse polymère est une mousse de polyuréthane présentant une densité comprise entre 0,10 g/cm³ et 0,30 g/cm³.

5. Prothèse mammaire interne (100) selon l'une quelconque des revendications précédentes, pourvue, entre l'arrière (103) et la partie avant (102), d'un bord qui s'amincit vers l'extérieur.

6. Prothèse mammaire interne (100) selon l'une des revendications 1 à 4, dans laquelle le corps (10) est pourvu d'une partie de fixation (120) qui constitue un support pour coudre la prothèse (100) directement sur le tissu musculaire.

7. Prothèse mammaire interne (100) selon la revendication 6, dans laquelle la partie de fixation (120) est une partie intégrante du corps (10), faite d'une seule pièce avec celui-ci et faite du même matériau.

8. Prothèse mammaire interne (100) selon la revendication 6 ou 7, dans laquelle la partie de fixation (120) est un bord annulaire continu (121) agencé autour du corps (10).

9. Prothèse mammaire interne (100) selon l'une des revendications précédentes, dans laquelle la partie de fixation (120) est un bord qui s'amincit vers l'extérieur, pour mieux suivre la forme naturelle de la glande mammaire et pour faciliter le positionnement de la prothèse sous le muscle.

10. Prothèse mammaire interne (100) selon la revendication 6 ou 7 ou 9, dans laquelle la partie de fixation (120) comprend une pluralité de fentes (122), réparties uniformément, chacune définissant un trou (124) pour le passage d'un fil de suture.

11. Prothèse mammaire interne (100) selon l'une des revendications précédentes, dans laquelle l'arrière (103) présente, en section transversale, une courbure (C1) obtenue par interpolation du point sternal (SP), du point de référence P_{1/3}, du point de référence P_{2/3} et du point thoracique latéral (PTL).

12. Prothèse mammaire interne (100) selon l'une des revendications précédentes, dans laquelle le revêtement est en silicone.

13. Procédé de fabrication d'une prothèse mammaire interne (100) selon la revendication 3, comprenant les étapes consistant à :
- préparer un moule comprenant une partie femelle et un couvercle, ladite partie femelle définissant au sein de celle-ci la cavité du moule ;
- combiner dans la cavité du moule, à température ambiante, les composants (A, B), et mélanger ;
- laisser la mousse de silicone s'expanser jusqu'à ce que le volume atteigne 2/3 du volume total de la cavité de moule, et ensuite fermer la cavité avec le couvercle.

14. Procédé de fabrication d'une prothèse mammaire interne (100) selon la revendication 13, dans lequel l'étape consistant à fournir un moule comprend les étapes consistant à :
a. collecter des données numériques sur la forme et les dimensions externes de la glande mammaire de la patiente au moyen d'un balayage de la glande mammaire ;
b. collecter des données numériques sur la taille et forme de la cage thoracique et de la glande mammaire de la patiente par tomodensitométrie (tomographie axiale commandée par ordinateur, ou TACO) ou micro-tomodensitométrie ;
c. traiter, à l'aide d'un logiciel, les données numériques collectées dans les étapes a) et b) pour définir, en trois dimensions, les forme, taille et volume de la cavité de moule.
